# EUROPEAN PATENT APPLICATION

(11) **EP 4 759 308 A1**
(43) Date of publication of application: **17.06.2026**
(21) Application number: 24383356.3
(22) Date of filing: 12.12.2024
(51) Int. Cl.: A61K 31/194, A61K 31/6615, A61K 36/38, A61P 13/00, A61P 13/04, A61P 13/12

(54) **COMBINATIONS OF HYDOXYCITRIC ACID AND PHYTIC ACID, OR THEIR PHARMACEUTICALLY ACCEPTABLE OR EDIBLE SALTS, USEFUL FOR THE TREATMENT OF CALCIUM DEPOSITION DISEASES**

(71) Applicant: Universitat de les Illes Balears, 07122 Palma de Mallorca (Islas Baleares) (ES)
(72) Inventor: GRASES FREIXEDAS, Feliciano, 07122 PALMA DE MALLORCA (ES); COSTA BAUZÀ, Antonia, 07122 PALMA DE MALLORCA (ES); DIETRICH TRIAS, Jaume, 07122 PALMA DE MALLORCA (ES)
(74) Representative: ZBM Patents - Zea, Barlocci & Markvardsen

(57) **Abstract**

The present disclosure relates to a combination of: a) hydroxycitric acid, or a pharmaceutically acceptable or an edible salt thereof, or, alternatively, a Garcinia plant extract which comprises hydroxycitric acid, or a pharmaceutically acceptable or an edible salt thereof, and b) phytic acid, or a pharmaceutically acceptable or an edible salt thereof, or, alternatively, a rice bran plant extract which comprises phytic acid, or a pharmaceutically acceptable or an edible salt thereof, in a molar ratio from 10:1 to 800:1, their use, in the treatment and/or prevention of conditions characterised by the pathological deposition of calcium oxalate crystals, as well as, to pharmaceutical compositions, edible products and kits containing them.

## Description

### Technical Field

The present invention relates to the field of medicine. In particular, it relates to compositions for the treatment, therapy or prevention of conditions characterised by the pathological deposition of calcium oxalate crystals. The compositions of the invention are particularly useful for the treatment of lithiasis.

### Background Art

Lithiasis is a medical condition characterised by the formation of calculi, often referred to as stones, in an organ or duct of the body. Said stones are hard, compact masses of material that form through the crystallisation of substances, typically mineral salts. Renal lithiasis (commonly known as kidney stones, regardless of their location in the urinary tract), occurs when such stones form anywhere in the urinary tract, with presence of stones in the kidneys being known as nephrolithiasis, whereas urolithiasis is typically used to refer to the presence of stones in the remainder of the urinary collecting system, including the ureters, bladder, and urethra. The prevalence of symptomatic renal lithiasis is estimated to be approximately 10% in the United States and Europe, but as high as 20% in Asian countries, with incidence on the rise. Currently, treatment options for symptomatic renal lithiasis patients largely focus on either expectant management, analgesic and anti-emetic medications, or surgical removal, in critical cases.

Approximately 70-80% of all kidney stones are composed primarily of calcium oxalate, with increased levels of urinary calcium and urinary oxalate being important risk factors for their development. Thus, compounds that form soluble complexes with calcium ions and/or with oxalate ions, significantly decrease calcium oxalate supersaturation in biological fluids, including urine, making it less likely that calcium oxalate crystals are generated. Such compounds belong to a wide class of compounds broadly referred to as crystallisation inhibitors.

Crystallisation inhibitors have already been proposed as a general therapeutic modality for the prevention and treatment of conditions characterised by the pathological deposition of calcium oxalate crystals, such as renal lithiasis, in the 1960s. Since then, many candidate molecules have been explored, ranging from small molecules - for example, magnesium, polyhydrocarboxylic acids (e.g.: tartronic acid, citric acid, hydroxycitric acid, hippuric acid, α-ketoglutaric acid), polyphosphates (e.g. phytic acid and analogues), vitamins (e.g. vitamins E, B1 and B6), natural polyphenols (e.g. quercetin, resveratrol) -, to macromolecules, including peptides, peptoids and synthetic polymers with carboxylic acid, sulphate, hydroxyl, and/or phosphate groups. For instance, Yang et al., in "Hydroxycitrate prevents calcium oxalate crystallisation and kidney injury in a nephrolithiasis rat model", Urolithiasis, 2022, volume 50, issue 1, pp 47-53, teaches that hydroxycitrate prevents calcium oxalate crystallisation, and Calvo et al., in "Effect of phytic acid (InsP6) and other inositol-phosphates (lnsP5, InsP4, InsP3, InsP2) on crystallisation of calcium oxalate, brushite, and hydroxyapatite", Biomolecules, 2023, volume 13, issue 7, pp 1061, discloses the effect of InsP2-InsP6 on calcium oxalate crystallisation. Despite the sustained efforts, over the past seven decades, to identify efficacious therapeutic approaches to prevent or treat the pathological crystallisation of calcium oxalate, to this date, the need remains to identify treatments with improved efficacy for the prevention and therapy of conditions characterised by the pathological crystallisation of calcium oxalate, in particular lithiasis. This is even more critical in view of the fact that conditions such as renal lithiasis are often recurring medical conditions: over half of the patients who have had kidney stones large enough to block the ureter and cause painful symptoms or other medical complications, are likely to develop them again within 10 years.

### Summary of Invention

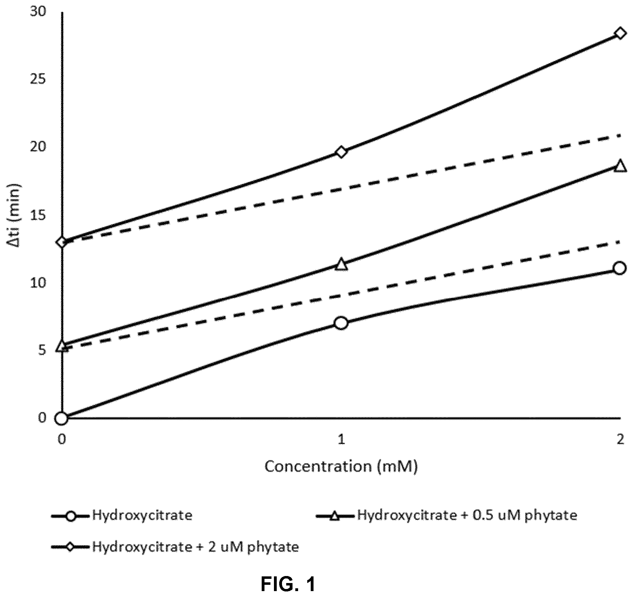

The inventors have surprisingly found that the combination of hydroxycitric acid and phytic acid, optionally in specific weight ratios, presents a synergistic inhibitory effect against the formation of calcium oxalate crystals (FIG. 1), thus providing a combination of improved efficacy for use in the treatment and/or prevention of conditions characterised by the pathological deposition of calcium oxalate crystals, including, but not limited to, lithiasis, in particular renal lithiasis.

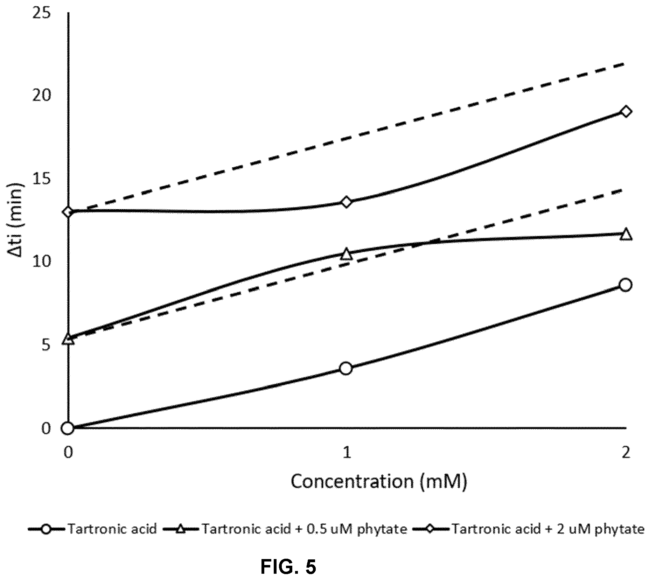

Remarkably, said synergistic effect was not observed when phytic acid was combined with other polyhydroxycarboxylic acids, such as tartronic acid (FIG. 5).

Moreover, said synergistic effect is all the more surprising in view of the fact that citrate, which is known to act via similar mechanisms to inhibit calcium oxalate crystallisation and which hydroxycitric acid is a structural analog of, does not show synergistic effects when combined with phytic acid, as disclosed by Grases et al., in "Efficacy of mixtures of magnesium, citrate and phytic acid as calcium oxalate crystallisation inhibitors in urine", 2015, J Urol, volume 194, issue 3, pp 812-819.

Therefore, although it is known that both hydroxycitric acid and phytic acid can individually inhibit calcium oxalate crystallisation, their combined therapeutic application as provided herein has not been previously proposed. Indeed, an expert, considering the prior art, would be dissuaded from employing such a combination at the specific weight ratios claimed.

Moreover, the vast majority of prior art disclosures regarding hydroxycitric acid have focused on its use in applications that are medically as well as mechanistically unrelated to lithiasis, specifically as a weight loss supplement due to its effects as an inhibitor of fatty acid synthesis and lipogenesis, and appetite suppressant.

Accordingly, a first aspect of the invention relates to a combination of: a) hydroxycitric acid, or a pharmaceutically acceptable or an edible salt thereof, or, alternatively, a Garcinia plant extract which comprises hydroxycitric acid, or a pharmaceutically acceptable or an edible salt thereof, and b) phytic acid, or a pharmaceutically acceptable or an edible salt thereof, or, alternatively, a rice bran plant extract which comprises phytic acid, or a pharmaceutically acceptable or an edible salt thereof; wherein: the molar ratio of hydroxycitric acid, or the pharmaceutically acceptable or the edible salt thereof, and phytic acid, or a pharmaceutically acceptable or an edible salt thereof, in the combination is from 10:1 to 800:1.

Furthermore, a second aspect of the invention relates to the combination of hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, as defined in the first and/or second aspects above, for use in the treatment and/or prevention of at least one condition characterised by the pathological deposition of calcium oxalate crystals in a mammal, wherein the treatment comprises administering a therapeutically effective amount of the combination.

Similarly, a third aspect of the invention relates to hydroxycitric acid, or a pharmaceutically acceptable or an edible salt thereof, or, alternatively, to a Garcinia plant extract which comprises hydroxycitric acid, or a pharmaceutically acceptable or an edible salt thereof, for use in the treatment and/or prevention of at least one condition characterised by the pathological deposition of calcium oxalate crystals in a mammal, in combination therapy with phytic acid, or a pharmaceutically acceptable or an edible salt thereof, or, alternatively, with a rice bran plant extract which comprises phytic acid, or a pharmaceutically acceptable or an edible salt thereof, wherein the treatment comprises administering a molar ratio from 10:1 to 800:1 of hydroxycitric acid, or the pharmaceutically acceptable or the edible salt thereof, and phytic acid, or the pharmaceutically acceptable or the edible salt thereof.

Conversely, a fourth aspect of the invention relates to phytic acid, or a pharmaceutically acceptable or an edible salt thereof, or, alternatively, to a rice bran plant extract which comprises phytic acid, or a pharmaceutically acceptable or an edible salt thereof, for use in the treatment and/or prevention of at least one condition characterised by the pathological deposition of calcium oxalate crystals in a mammal, in combination therapy with hydroxycitric acid, or a pharmaceutically acceptable or an edible salt thereof, or, alternatively, with a Garcinia plant extract which comprises hydroxycitric acid, or a pharmaceutically acceptable or an edible salt thereof, wherein the treatment comprises administering a molar ratio from 10:1 to 800:1 of hydroxycitric acid, or the pharmaceutically acceptable or the edible salt thereof, and phytic acid, or the pharmaceutically acceptable or the edible salt thereof.

A fifth aspect of the invention provides a pharmaceutical composition comprising a therapeutically effective amount of the combination of the invention, as defined in the first and/or second aspects above, together with pharmaceutical excipients or carriers.

A further, sixth, aspect of the invention provides an edible product comprising a therapeutically effective amount of the combination of the invention, as defined in the first and/or second aspects above.

In a seventh aspect, the invention provides a kit comprising: (a) a pharmaceutical composition comprising a therapeutically effective amount of hydroxycitric acid, or a pharmaceutically acceptable or an edible salt thereof, or, alternatively, a Garcinia plant extract which comprises hydroxycitric acid, or a pharmaceutically acceptable or an edible salt thereof, together with pharmaceutical excipients or carriers; (b) a pharmaceutical composition comprising a therapeutically effective amount of phytic acid, or a pharmaceutically acceptable or an edible salt thereof, or, alternatively, a rice bran plant extract which comprises phytic acid, or a pharmaceutically acceptable or an edible salt thereof, together with pharmaceutical excipients or carriers; and (c) optionally, instructions for their administration; wherein: the amounts to be administered of hydroxycitric acid, or the pharmaceutically acceptable or the edible salt thereof, and of phytic acid, or the pharmaceutically acceptable or the edible salt thereof, are such that the molar ratio of the hydroxycitric acid, or the pharmaceutically acceptable or the edible salt thereof, and the phytic acid, or the pharmaceutically acceptable or the edible salt thereof, is from 10:1 to 800:1.

### Brief Description of Drawings

FIG. 1 shows the effects of hydroxycitric acid+phytic acid mixtures on the inhibition of the precipitation of calcium oxalate crystals observed for different concentrations of hydroxycitric acid (as indicated on the x-axis in milimolar) in combination with 0 (circles), 0.5 micromolar (triangles), or 2 micromolar (diamonds) phytic acid, respectively. Dashed lines indicate the predicted values that would be expected to be observed if the effects of hydroxycitric acid + phytic acid were additive. Bottom dashed line indicates the predicted values if the effects of hydroxycitric acid + 0.5 micromolar phytic acid were additive. Top dashed line indicates the predicted values if the effects of hydroxycitric acid + 2 micromolar phytic acid were additive. Plotted on the y-axis is the increment of induction time.
FIG. 2 shows scanning electron microscopy photograph of calcium oxalate crystals formed in the presence of 5 milimolar hydroxycitric acid. Crystals were collected by filtration 30 min after induction time.
FIG. 3 shows scanning electron microscopy photograph of calcium oxalate crystals formed in the presence of 1 micromolar phytic acid. Crystals were collected by filtration 30 min after induction time.
FIG. 4 shows scanning electron microscopy photograph of calcium oxalate crystals formed in the presence of a combination of 2 milimolar hydroxycitric acid and 1 micromolar phytic acid. Crystals were collected by filtration 30 min after induction time.
FIG. 5 shows the effects of phytic acid + tartronic acid mixtures on calcium oxalate crystallisation. Solid lines show effects observed for different concentrations of tartronic acid (as indicated on the x-axis in milimolar) in combination with 0 (circles), 0.5 micromolar (triangles), or 2 micromolar (diamonds) phytic acid, respectively. Dashed lines indicate the predicted values that would be expected to be observed if the effects of tartronic acid + phytic acid were additive. Bottom dashed line indicates the predicted values if the effects of tartronic acid + 0.5 micromolar phytic acid were additive. Top dashed line indicates the predicted values if the effects of tartronic acid + 2 micromolar phytic acid were additive. Plotted on the y-axis is the increment of induction time.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. As used herein, the indefinite articles "a" and "an" are synonymous with "at least one" or "one or more." Unless indicated otherwise, definite articles used herein, such as "the" also include the plural of the noun.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention.

The term "weight ratio", as used herein, refers to the relation of weights of the molecules or compounds indicated. For instance, a hydroxycitric acid:phytic acid weight ratio (weightweight) of 50:1 refers to 50 mass units of hydroxycitric acid for every 1 mass unit of phytic acid, such as, for example, 50 mg hydroxycitric acid to 1 mg phytic acid. As used herein, "% by weight" or "% w/w" of a component refers to the amount of the single component relative to the total weight of the composition or, if specifically mentioned, of another component. In the present invention the weight of an extract refers to the dry extract weight or mass.

The term "molar ratio", as used herein, refers to the ratio between the amounts in moles of the molecules or compounds indicated. For instance, a hydroxycitric acid:phytic acid molar ratio (amount in moles:amount in moles) of 50:1 refers to 50 moles of hydroxycitric acid for every 1 mole of phytic acid.

Throughout the description and claims, any weight ratio or mass relate to the weight ratio or mass corresponding to those of free hydroxycitric acid and/or free phytic acid, respectively. The skilled person understands that where hydroxycitric acid and/or phytic acid are in the form of a salt thereof, the mass or weight ratio of hydroxycitric acid salt and/or phytic acid salt need to be adjusted accordingly.

As used herein, the terms "inhibiting" in the context of the effect of compounds on the crystallisation of calcium oxalate refers to lowering the rate of, delaying, slowing down, preventing or blocking calcium oxalate crystallisation, as well as reducing the size, absolute number or relative number of calcium oxalate crystals.

"Simultaneous administration", as used herein, refers to the co-administration of hydroxycitric acid, or a pharmaceutically acceptable or an edible salt thereof, and phytic acid, or a pharmaceutically acceptable or an edible salt thereof, either at the same time or virtually at the same time, such as within a very short period of time of each other, such as within less than 10 minutes of each other.

"Sequential administration", as used herein, refers to the administration of hydroxycitric acid, or a pharmaceutically acceptable or an edible salt thereof, and phytic acid, or a pharmaceutically acceptable or an edible salt thereof, wherein the administration of hydroxycitric acid, or of the pharmaceutically acceptable or the edible salt thereof, occurs in a specific order, either before or after the administration of phytic, or of the pharmaceutically acceptable or the edible salt. The time interval between the administration of hydroxycitric acid, or of the pharmaceutically acceptable or the edible salt thereof, and the administration of phytic, or of the pharmaceutically acceptable or the edible salt thereof, is a predetermined time interval.

"Separate administration", as used herein, refers to the administration of hydroxycitric acid, or a pharmaceutically acceptable or an edible salt thereof, at separate points in time from the administration of phytic acid, or a pharmaceutically acceptable or an edible salt. The time interval between the administration of hydroxycitric acid, or of the pharmaceutically acceptable or the edible salt thereof, and the administration of phytic, or of a pharmaceutically acceptable or the edible salt thereof, may be from 6 hours to 48 hours, particularly between 6 hours to 24 hours.

"Pharmaceutical composition", as used herein, refers to a composition suitable for pharmaceutical administration that comprises at least one pharmaceutically active ingredient and one or more pharmaceutically acceptable carriers, diluents or excipients.

The term "dosage form" or dosage unit" refers to a pharmaceutical entity that comprises an active pharmaceutical ingredient and pharmaceutically acceptable excipients or carriers, and which is to be administered to, or to be taken by a mammal, including a human. The term "pharmaceutical dosage form" or "pharmaceutical dosage unit" also encompasses non-reusable packaging as well, especially when a pharmaceutical composition is individually packaged.

The term "pharmaceutically acceptable" refers to a compound, excipient or carrier that is chemically and/or toxicologically compatible with other ingredients constituting a preparation, and is suitable for use in the preparation of compositions for administration to mammals, in particular humans. Thus, such a "pharmaceutically acceptable" compound, excipient or carrier is suitable for use in contact with a tissue or organ of humans and non-human animals without excessive toxicity, irritation, allergic response, immunogenicity or other problems or complications commensurate with a reasonable benefit/risk ratio.

By "pharmaceutically acceptable excipient" it should be understood as any pharmaceutically acceptable conventional vehicle used in the manufacture of pharmaceutical compositions. Depending on the formulation, these include, but are not limited to, inert diluents, fillers or bulking agents, solvents, co-solvents, dispersing and/or granulating agents, viscosity modifying agents, suspending agents, surfactants, surface active agents and/or emulsifiers, stabilizers, disintegrating agents, binders, adhesives, preservatives, antioxidants, opacifiers, coating agents, buffering agents, wetting agents, glidants or lubricants, oils, complexing agents, chelating agents, tonicity agents, and combinations thereof. Excipients such as coloring agents, coating agents, sweetening agents, and flavoring agents can be present in the composition, according to the judgment of the formulator.

The term "pharmaceutically acceptable or edible salt" refers to a relatively non-toxic addition salt of a compound of the present disclosure. For example, see Berge et al. "Pharmaceutical Salts", 1997, J Pharm Sci, volume 66, issue 1, pp 1-19.

Suitable pharmaceutically acceptable or edible salts of the compounds of the present disclosure may be, for example, but not limited to, the sodium salts, potassium salts, calcium salts, magnesium salts and zinc salts.

The present invention includes all possible salts of each of the compounds of the present invention, namely hydroxycitric acid and phytic acid, which may be a single salt or any mixture of the salts in any ratio.

The term "subject" or "patient" in the present application includes humans and non-human mammals.

A "food composition", as used herein, refers to any solid, semi-solid, or liquid composition, also a drinkable liquid, which is suitable for human consumption.

The term "animal feed composition" refers to any solid, semi-solid, or liquid composition, also a drinkable liquid, which is suitable for non-human animal consumption; therefore, it also encompasses pet food.

Both food and feed compositions are meant to also encompass the raw materials and feedstock used for their elaboration, as well as dietary supplements.

The term "dietary supplement" refers to a preparation intended to supplement the diet and provide nutrients, such as vitamins, minerals, fiber, fatty acids, or amino acids, that may be missing or may not be consumed in sufficient quantity in a subject's diet.

The term "edible" as used herein refers to a substance that can be ingested by animals, including humans, without significant deleterious health consequences.

The term "plant extract" as used herein refers to concentrated preparation of compounds obtained from plants or parts of plants.

In the present invention, "hydroxycitric acid" is understood as a molecule identified by CAS numbers: 27750-10-3 or 6205-14-7, of the formula (I): 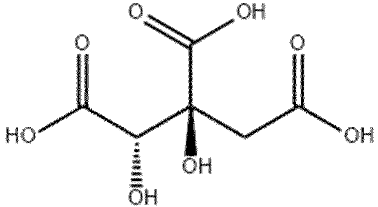 and its pharmaceutically acceptable or edible salts, which include but are not limited to sodium, potassium, calcium, magnesium, zinc, calcium-magnesium, potassium-sodium, sodium-calcium, calcium-potassium-magnesium, calcium-potassium-zinc salts or combinations thereof. For the purposes of the present invention, hydroxycitric acid, or the pharmaceutically acceptable or the edible salts thereof, may be used in free form as pure substances, extracts of plant species containing them, such as, for example, extracts of plant parts from Garcinia species plants such as Garcinia atroviridis, Garcinia indica and Garcinia cowa, as well as in the fruits, leaves and calyxes of Hibiscus sabdariffa plants, in particular extracts from the fruit rinds of Garcinia cambogia or from the calyx of Hibiscus sabdariffa.

By "Garcinia plant extract" it is understood a concentrated preparation of compounds obtained from parts of plants of the genus Garcinia. Similarly, by "Garcinia cambogia plant extract" and "Hibiscus sabdariffa plant extract" it is understood a concentrated preparation of compounds obtained from parts of the Garcinia cambogia (also known as Garcinia gummi-gutta) and Hibiscus sabdariffa plants, respectively. In the present invention, it is particular to use the rind of the dried fruit or leaves of Garcinia cambogia, or the calyx of Hibiscus sabdariffa, to prepare the extract.

In humans and mammals, hydroxycitric acid is excreted in urine at a rate of approximately 10-20% of the amount an orally ingested dose (Chung et al., "Molecular modifiers reveal a mechanism of pathological crystal growth inhibition", Nature, 2016, volume 536, pp 446-450). Unless ingested from exogenous sources, hydroxycitric acid is not otherwise naturally present in human urine.

The stereoisomers of hydroxycitric acid are related structurally to citric acid wherein a hydroxy group is substituted for one of the four methylene hydrogens. Thus, there are four possible stereoisomers of hydroxycitric acid. Of these, (-)-hydroxycitric acid is the one found in fruit rind of Garcina sp. plants and, thus, the most commonly used.

Hydroxycitric acid is commercially available and can be obtained from several chemical suppliers, for example SigmaAldrich or Merck.

Alternatively, hydroxycitric acid can be obtained by isolation from the fruit of Garcinia species using known procedures, for example: Lewis "Methods in Enzymology", J. M. Lowenstein, Ed., volume 13, pp. 613-623, Academic Press, New York, 1969, or by isolation from the calyx of Hibiscus sabdariffa by using methods known in the art, for example: Singh et al. "Extraction and characterization of polyphenolic compounds and potassium hydroxycitrate from Hibiscus sabdariffa", 2021, Future Foods, Volume 4, 100087.

Hydroxycitric acid may also be prepared synthetically, for example starting from citric acid as disclosed in WO0000188 A2.

Phytic acid (also known as inositol hexakisphosphate, myo-inositol hexaphosphate, myo-Inositol-1,2,3,4,5,6-hexakisphosphate, InsP6 or IP6), is a naturally occurring storage form of phosphorus in plants. Natural sources high in phytic acid are plant seeds and/or grains (mainly cereals), legumes (e.g. beans) and nuts, although it is also found, albeit in smaller quantities, in other plant parts, including pollen, roots, tubers, turions and fruits. Plant phytic acid is present primarily as a salt of mono- and divalent cations (Mg²⁺, K⁺, Ca²⁺, Zn²⁺, Fe²⁺, and Cu²⁺). The majority of phytic acid in animal tissues and organs is of dietary origin.

Most of phytic acid, or of a pharmaceutically acceptable or an edible salt thereof, ingested from exogenous/dietary sources is degraded in the gastrointestinal tract. Consequently, the excretion rate of phytic acid in urine is from approximately 0.5% to 2% of the amount of an orally ingested dose.

In the present invention, phytic acid' or 'myo-inositol-hexaphosphate' is understood as the molecule identified by CAS number: 83-86-3, of the formula (II): 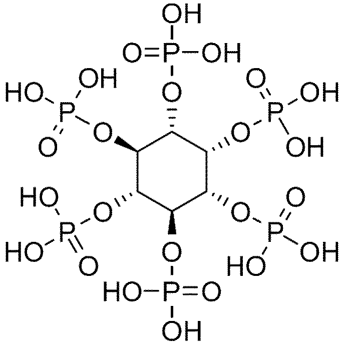 and its pharmaceutically acceptable or edible salts, which include but are not limited to sodium, potassium, calcium, magnesium, zinc and calcium-magnesium salts. For the purposes of the present invention, phytic acid, and/or its pharmaceutically acceptable or edible salts, may be used in free form as pure substances, in the form of extracts of plant species containing them (such as, for example, extracts of brown rice), in the form of plant parts (such as the germs, the external parts, grains or fruits, including nuts, of such plants) from plant species containing them (for example, maize, brown rice, wild rice, lentils, wheat, oat, barley, sorghum, rye, millet, soy, locust bean, cacao, almond, walnut, peanut, hazelnut, Brazil nut etc).

Phytic acid is commercially available and can be obtained from several chemical suppliers, for example SigmaAldrich or Merck.

Alternatively, phytic acid can be extracted from suitable plant sources, such as rice bran or beans, by methods known in the art, for example: Canan et al., "Studies on the extraction and purification of phytic acid from rice bran", J Food Comp Analysis, 2011, volume 24, issue 7, pp 1057-1063. By "rice bran plant extract" it is understood a concentrated preparation of compounds obtained from the outer layer of rice grains known as rice bran.

It would be evident for the skilled in the art that if hydroxycitric acid, or the pharmaceutically acceptable or the edible salt thereof, and/or phytic acid, or the pharmaceutically acceptable or the edible salt thereof, are provided in the form of plant extracts, the mass amounts of the extracts should be adjusted depending on the amount of hydroxycitric acid and/or phytic acid contained in the respective extracts to obtain the mass amounts or concentrations of hydroxycitric acid and/or phytic acid of the present invention.

Similarly, it is also evident for the skilled in the art that if hydroxycitric acid, or the pharmaceutically acceptable or the edible salt thereof, and/or phytic acid, or the pharmaceutically acceptable or the edible salt thereof, are provided in the form of plant extracts, the weight ratios of the extracts should be adjusted depending on the amount of hydroxycitric acid and/or phytic acid contained in the respective extracts to obtain the weight ratios or molar ratios of hydroxycitric acid to phytic acid of the present invention.

For establishing the effects of the compounds of the combination of the present invention on calcium oxalate crystallisation, a turbidimetric assay in synthetic urine method was employed, as adapted from the method disclosed, for example, by Grases et al., in "Efficacy of mixtures of magnesium, citrate and phytic acid as calcium oxalate crystallisation inhibitors in urine", 2015, J Urol, volume 194, issue 3, pp 812-819, as further illustrated in the examples of the invention.

A synergistic effect is considered to be present when the effect observed for a combination of two or more compounds is greater than the sum of the effects observed for each of the compounds individually. Thus, a synergistically effective combination is considered to be a combination of two or more compounds that provides a synergistic effect, when the effect is assessed using suitable in vitro assays. In the context of the present invention, suitable in vitro assays include, but are not limited to, those described in the previous paragraph and those disclosed in the practical examples disclosed herein.

The term 'calcifications' or 'stones' includes all such process or conditions which imply/induce the formation of solid precipitates in the urine or the urinary system (e.g. kidneys, ureters, bladder, uretra). Thus, the conditions that induce calcifications or kidney stones include, but are not limited to, a high level of urinary oxalate, a high level in the urinary excretion of calcium or a reduced presence or activity of nucleation and crystal growth inhibitors.

Hypercalciuria refers to an elevated urinary calcium excretion which exceeds 300 mg/24 hours, in adult humans, or 4 mg/kg body weight/24 hours.

Normal oxalate excretion levels in adult human urine range from 8 to 43 mg per 24-hour period, with the average excretion being slightly higher in men than in women. Therefore, a urinary oxalate concentration of up to approximately 20 to 22 mg oxalate/1000 mL urine is generally considered normal.

Hyperoxaluria refers to a clinical condition characterised by urinary oxalate excretion that exceeds 45 mg/24 hours in adult humans. The levels of oxalate in the urine can reach levels of 90 up to 270 mg/24 hours in patients with primary hyperoxaluria. Hyperoxaluria is known to be a risk factor for pathological calcium oxalate crystal deposition in the urinary tract, and kidney stone formation.

Hypocitraturia refers to a metabolic abnormality characterised by low citrate levels in the urine. In adult humans, hypocitraturia is generally defined as a urinary citrate excretion below 320 mg/24 hours. Under this definition, severe hypocitraturia refers to a urinary citrate excretion rate of less than 100 mg/24 hours, while a urinary citrate excretion rate of between 100 to 320 mg/24 hours is classified as mild to moderate hypocitraturia.

As referred herein, a combination according to the present invention comprises hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, in certain weight or molar ratios.

Similarly, a pharmaceutical composition, pharmaceutical product, pharmaceutical combination, kit, foodstuff product, drink product, dietary supplement product for human consumption, animal feed product, veterinary product and dietary supplement product for non-human animal consumption, according to the present invention, comprises hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, optionally in therapeutically effective amounts and in certain weight or molar ratios.

In a particular embodiment, optionally in combination with any of the particular embodiments described above or below throughout the description, hydroxycitric acid, or the pharmaceutically acceptable or the edible salt thereof, is (-)-hydroxycitric acid, or a pharmaceutically acceptable or an edible salt thereof.

In a particular embodiment, optionally in combination with any of the particular embodiments described above or below throughout the description, the Garcinia plant extract which comprises hydroxycitric acid, or the pharmaceutically acceptable or the edible salt thereof, is a Garcinia plant extract which comprises (-)-hydroxycitric acid, or a pharmaceutically acceptable or an edible salt thereof.

The combinations and pharmaceutical compositions of the present invention are suitable for administration in mammals, including humans. Similarly, the edible compositions of the present invention are suitable for consumption by mammals, including humans.

The pharmaceutical compositions comprising the combination of the present invention can be presented in any dosage form, for which they will include the pharmaceutically acceptable excipients necessary for the formulation of the desired dosage form. Dosage forms include, but are not limited to, solid, semi-solid, gel, powder and liquid dosage forms.

The pharmaceutical compositions of the present invention may comprise hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, in a combined single dosage form, for simultaneous administration.

Alternatively, the combination of the invention may be formulated as separate dosage forms: a first dosage form comprising hydroxycitric acid, or a pharmaceutically acceptable or an edible salt thereof, or, alternatively, a Garcinia plant extract which comprises hydroxycitric acid, or a pharmaceutically acceptable or an edible salt thereof, and a second dosage form comprising phytic acid, or a pharmaceutically acceptable or an edible salt thereof, or, alternatively, a rice bran plant extract which comprises phytic acid, or a pharmaceutically acceptable or an edible salt thereof, wherein the separate dosage forms may be administered simultaneously, sequentially (in any order) or separately (in any order).

If formulated as separate dosage forms, said separate dosage forms can optionally be co-packaged, for example in a single container or in a plurality of containers within a single outer package, or co-presented in separate packaging ("common presentation"). As an example of co-packaging or common presentation, a kit is contemplated comprising, in separate containers, hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt, or, alternatively, a rice bran plant extract which comprises phytic acid, or a pharmaceutically acceptable or an edible salt thereof. In another example, hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, are separately packaged and available for sale independently of one another, but are co-marketed or co-promoted for use according to the invention. The separate dosage forms may also be presented to a subject separately and independently, for use according to the invention.

The combination of the present invention and the pharmaceutical compositions comprising combination of the present invention may be administered by any suitable route, in particular, parenteral or enteral, including oral. If formulated as separate dosage forms, the separate dosage forms may be administered by the same or different routes. The skilled person is familiar with methods of formulating pharmaceutical compositions suitable for different modes of administration.

Enteral administration delivers pharmaceutically active substances to the body through the gastrointestinal tract. Appropriate modes of enteral administration include, but are not limited to, oral, rectal, gastrointestinal, sublingual and buccal administration, or combinations thereof.

Parenteral administration includes any suitable route of administration, other than topical, where a pharmaceutically active substance is delivered to the body via routes other than the gastrointestinal tract. Appropriate modes of parenteral administration routes include, but are not limited to, intravenous, urethral, subcutaneous, subdermal, transdermal, or intramuscular, or combinations thereof.

Unlike topical administration, where a pharmaceutically active substance is delivered locally, in proximity to the target site of action, enteral and parenteral administration routes allow the systemic delivery of pharmaceutically active substances into the body.

As mentioned above belonging to the present invention, is a combination of: a) hydroxycitric acid, or a pharmaceutically acceptable or an edible salt thereof, or, alternatively, a Garcinia plant extract which comprises hydroxycitric acid, or a pharmaceutically acceptable or an edible salt thereof, and b) phytic acid, or a pharmaceutically acceptable or an edible salt thereof, or, alternatively, a rice bran plant extract which comprises phytic acid, or a pharmaceutically acceptable or an edible salt thereof, wherein the molar ratio of hydroxycitric acid, or a pharmaceutically acceptable or an edible salt thereof, and phytic acid, or a pharmaceutically acceptable or an edible salt thereof, in the combination is from 10:1 to 800:1.

The combination of the present invention may also be more precisely defined as a combination of: a) hydroxycitric acid, or a pharmaceutically acceptable or an edible salt thereof, or, alternatively, a Garcinia plant extract which comprises hydroxycitric acid, or a pharmaceutically acceptable or an edible salt thereof, and b) phytic acid, or a pharmaceutically acceptable or an edible salt thereof, or, alternatively, a rice bran plant extract which comprises phytic acid, or a pharmaceutically acceptable or an edible salt thereof, wherein the molar ratio of a) and b) in the combination is such that, when the combination is administered to a subject, it results in: (i) a concentration of phytic acid, or of a pharmaceutically acceptable or an edible salt thereof, in urine from 0.5 micromolar to 5 micromolar of phytic acid, within 20 days post-administration; and (ii) a molar ratio of hydroxycitric acid, or the pharmaceutically acceptable or the edible salt thereof, and phytic acid, or the pharmaceutically acceptable or the edible salt thereof, in urine from 500:1 to 8,000:1, expressed as a ratio of the amount, in milimoles, of hydroxycitric acid, or the pharmaceutically acceptable or the edible salt thereof, per litre of urine, to the amount, in milimoles, of phytic acid, or the pharmaceutically acceptable or the edible salt thereof, per litre of urine; wherein the concentration of hydroxycitric acid, or the pharmaceutically acceptable or the edible salt thereof, in urine is measured by ion chromatography, and the concentration of phytic acid, or the pharmaceutically acceptable or the edible salt thereof, in urine is measured by inductively coupled plasma atomic emission spectrometry.

In a more particular embodiment of the combination as defined in the previous paragraph, optionally in combination with any of the particular embodiments described above or below throughout the description, the molar ratio of a) and b) in the combination is such that, when the combination is administered to a subject, it results in: (i) a concentration of phytic acid, or of a pharmaceutically acceptable or an edible salt thereof, in urine from 0.5 micromolar to 2 micromolar of phytic acid, within 20 days post-administration; and (ii) a molar ratio of hydroxycitric acid, or the pharmaceutically acceptable or the edible salt thereof, and phytic acid, or the pharmaceutically acceptable or the edible salt thereof, in urine from 500:1 to 4,000:1, expressed as a ratio of the amount, in milimoles, of hydroxycitric acid, or the pharmaceutically acceptable or the edible salt thereof, per litre of urine, to the amount, in milimoles, of phytic acid, or the pharmaceutically acceptable or the edible salt thereof, per litre of urine; wherein the concentration of hydroxycitric acid, or the pharmaceutically acceptable or the edible salt thereof, in urine is measured by ion chromatography, and the concentration of phytic acid, or the pharmaceutically acceptable or the edible salt thereof, in urine is measured by inductively coupled plasma atomic emission spectrometry.

Methods suitable for determining the concentration of hydroxycitric acid, or the pharmaceutically acceptable or edible salts thereof, in urine, including human urine, are known in the art, for example, ion chromatography, as disclosed in Chung et al., "Molecular modifiers reveal a mechanism of pathological crystal growth inhibition", Nature, 2016, volume 536, pp 446-450.

Similarly, methods suitable for determining the concentration of phytic acid, or the pharmaceutically acceptable or edible salts thereof, in urine, including human urine, are known in the art, such as, for example, inductively coupled plasma atomic emission spectrometry, as disclosed by Grases et al. "Determination of myo-inositol hexakisphosphate (phytate) in urine by inductively coupled plasma atomic emission spectrometry", 2004, Analytica Chimica Acta, vol. 510, issue 1, pp. 41-43.

In a particular embodiment of the combination of the invention, as defined above, optionally in combination with any of the particular embodiments described above or below throughout the description, the molar ratio of hydroxycitric acid, or the pharmaceutically acceptable or the edible salt thereof, and phytic acid, or the pharmaceutically acceptable or the edible salt thereof, in the combination is from 15:1 to 700:1, particularly from 20:1 to 600:1, particularly from 25:1 to 500:1, particularly from 30:1 to 400:1, particularly from 35:1 to 300:1, particularly from 40:1 to 200:1, particularly from 45:1 to 100:1, particularly from 50:1 to 75:1.

In another particular embodiment of the combination of the invention, as defined above, optionally in combination with any of the particular embodiments described above or below throughout the description, the molar ratio of hydroxycitric acid, or the pharmaceutically acceptable or the edible salt thereof, and phytic acid, or the pharmaceutically acceptable or the edible salt thereof, in the combination is from 12:1 to 400:1, particularly from 14:1 to 300:1, particularly from 16:1 to 200:1, particularly from 18:1 to 100:1, particularly from 20:1 to 90:1, particularly from 22:1 to 80:1, particularly from 24:1 to 70:1, particularly from 26:1 to 60:1.

In another particular embodiment of the combination of the invention, as defined above, optionally in combination with any of the particular embodiments described above or below throughout the description, the molar ratio of hydroxycitric acid, or the pharmaceutically acceptable or the edible salt thereof, and phytic acid, or the pharmaceutically acceptable or the edible salt thereof, in the combination is from 10:1 to 300:1, particularly from 12:1 to 240:1, particularly from 15:1 to 200:1, particularly from 18:1 to 180:1, particularly from 21:1 to 150:1, particularly from 24:1 to 120:1, particularly from 27:1 to 90:1, particularly from 30:1 to 60:1. In a more particular embodiment, the molar ratio of hydroxycitric acid, or the pharmaceutically acceptable or the edible salt thereof, and phytic acid, or the pharmaceutically acceptable or the edible salt thereof, in the combination is from 15:1 to 60:1.

In another particular embodiment of the combination of the invention, as defined above, optionally in combination with any of the particular embodiments described above or below throughout the description, the weight ratio of hydroxycitric acid, or the pharmaceutically acceptable or the edible salt thereof, and phytic acid, or the pharmaceutically acceptable or the edible salt thereof, in the combination is from 2:1 to 200:1, particularly from 3:1 to 150:1, particularly from 4:1 to 100:1, particularly from 5:1 to 75:1, particularly from 6:1 to 50:1, particularly from 7:1 to 40:1, particularly from 8:1 to 35:1, particularly from 9:1 to 30:1, particularly from 10:1 to 25:1, particularly from 15:1 to 20:1, expressed as a ratio of the mass corresponding to hydroxycitric acid to the mass corresponding to phytic acid.

In another particular embodiment of the combination of the invention, as defined above, optionally in combination with any of the particular embodiments described above or below throughout the description, the weight ratio of hydroxycitric acid, or the pharmaceutically acceptable or the edible salt thereof, and phytic acid, or the pharmaceutically acceptable or the edible salt thereof, in the combination is from 3:1 to 90:1, particularly from 4:1 to 80:1, particularly from 5:1 to 70:1, particularly from 6:1 to 60:1, particularly from 7:1 to 50:1, particularly from 8:1 to 40:1, particularly from 9:1 to 30:1, particularly from 10:1 to 20:1, expressed as a ratio of the mass corresponding to hydroxycitric acid to the mass corresponding to phytic acid.

In another particular embodiment of the combination of the invention, as defined above, optionally in combination with any of the particular embodiments described above or below throughout the description, the weight ratio of hydroxycitric acid, or the pharmaceutically acceptable or the edible salt thereof, and phytic acid, or the pharmaceutically acceptable or the edible salt thereof, in the combination is from 2:1 to 50:1, particularly from 3:1 to 45:1, particularly from 4:1 to 40:1, particularly from 5:1 to 35:1, particularly from 6:1 to 30:1, particularly from 7:1 to 25:1, particularly from 8:1 to 20:1, particularly from 9:1 to 15:1, particularly from 10:1 to 14:1, particularly from 11:1 to 13:1, expressed as a ratio of the mass corresponding to hydroxycitric acid to the mass corresponding to phytic acid.

In another particular embodiment of the combination of the invention, as defined above, optionally in combination with any of the particular embodiments described above or below throughout the description, the weight ratio of hydroxycitric acid, or the pharmaceutically acceptable or the edible salt thereof, and phytic acid, or the pharmaceutically acceptable or the edible salt thereof, in the combination is from 2:1 to 12:1, particularly from 3:1 to 11:1, particularly from 4:1 to 10:1, particularly from 5:1 to 9:1, particularly from 6:1 to 8:1, expressed as a ratio of the mass corresponding to hydroxycitric acid to the mass corresponding to phytic acid. In a more particular embodiment, the weight ratio of hydroxycitric acid, or the pharmaceutically acceptable or the edible salt thereof, and phytic acid, or the pharmaceutically acceptable or the edible salt thereof, in the combination is from 6:1 to 8:1, expressed as a ratio of the mass corresponding to hydroxycitric acid to the mass corresponding to phytic acid.

In another particular embodiment of the combination of the invention, as defined above, optionally in combination with any of the particular embodiments described above or below throughout the description, the weight ratio of hydroxycitric acid, or the pharmaceutically acceptable or the edible salt thereof, and phytic acid, or the pharmaceutically acceptable or the edible salt thereof, in the combination is from 2:1 to 50:1, more particularly from 2:1 to 45:1, more particularly from 2:1 to 40:1, more particularly from 2:1 to 35:1, more particularly from 2:1 to 30:1, more particularly from 2:1 to 25:1, more particularly from 2:1 to 20:1, more particularly from 2:1 to 15:1, more particularly from 2:1 to 10:1, more particularly from 2:1 to 9:1, more particularly from 2:1 to 8:1, more particularly from 2:1 to 7:1, more particularly from 2:1 to 6:1, more particularly from 2:1 to 5:1, more particularly from 2:1 to 4:1, expressed as a ratio of the mass corresponding to hydroxycitric acid to the mass corresponding to phytic acid.

In another particular embodiment of the combination of the invention, as defined above, optionally in combination with any of the particular embodiments described above or below throughout the description, hydroxycitric acid, or the pharmaceutically acceptable or the edible salt thereof, is (-)-hydroxycitric acid, or a pharmaceutically acceptable or an edible salt thereof.

In an alternative particular embodiment of the combination of the invention, as defined above, optionally in combination with any of the particular embodiments described above or below throughout the description, the Garcinia plant extract which comprises hydroxycitric acid, or the pharmaceutically acceptable or the edible salt thereof, is a Garcinia plant extract which comprises (-)-hydroxycitric acid, or a pharmaceutically acceptable or an edible salt thereof.

In another particular embodiment of the combination of the invention, as defined above, optionally in combination with any of the particular embodiments described above or below throughout the description, the pharmaceutically acceptable or the edible salts of hydroxycitric acid are selected from sodium hydroxycitrate, potassium hydroxycitrate, calcium hydroxycitrate, magnesium hydroxycitrate, zinc hydroxycitrate, calcium-magnesium hydroxycitrate, potassium-sodium hydroxycitrate, sodium-calcium hydroxycitrate, calcium-potassium-magnesium hydroxycitrate, calcium-potassium-zinc hydroxycitrate, and combinations thereof.

In another particular embodiment of the combination of the invention, as defined above, optionally in combination with any of the particular embodiments described above or below throughout the description, the pharmaceutically acceptable or the edible salts of phytic acid are selected from sodium phytic acid, potassium phytic acid, calcium phytic acid, magnesium phytic acid, zinc phytic acid, calcium-magnesium phytic acid, and combinations thereof.

In another particular embodiment of the combination of the invention, as defined above, optionally in combination with any of the particular embodiments described above or below throughout the description, phytic acid, or the pharmaceutically acceptable or the edible salt thereof, is provided in the form of a plant extract from a plant species or part of a plant species rich in phytic acid or its salts. In a particular embodiment, the plant extract from a plant species or part of a plant species rich in phytic acid or its salts is a rice bran plant extract.

In another particular embodiment of the combination of the invention, as defined above, optionally in combination with any of the particular embodiments described above or below throughout the description, hydroxycitric acid, or the pharmaceutically acceptable or the edible salt thereof, is provided in the form of a plant extract from a plant species or part of a plant species rich in hydroxycitric acid or its salts. In a particular embodiment, the plant extract from a plant species or part of a plant species rich in hydroxycitric acid or its salts is a Garcinia plant extract. In a further particular embodiment, the Garcinia plant extract is a Garcinia cambogia plant extract. In a yet further particular embodiment, the Garcinia cambogia plant extract is a plant extract prepared from the fruit of a Garcinia cambogia plant. In another yet further particular embodiment, the plant extract prepared from the fruit of a Garcinia cambogia plant is prepared from the dry fruit of a Garcinia cambogia plant.

In another particular embodiment of the combination of the invention, as defined above, optionally in combination with any of the particular embodiments described above or below throughout the description, hydroxycitric acid, or the pharmaceutically acceptable or the edible salt thereof, is provided in the form of a plant extract selected from a group consisting of: a Garcinia cambogia plant extract, a Hibiscus sabdariffa plant extract, and combinations thereof.

In another particular embodiment of the combination of the invention, as defined above, optionally in combination with any of the particular embodiments described above or below throughout the description, hydroxycitric acid, or the pharmaceutically acceptable or the edible salt thereof, is provided in the form of a Garcinia cambogia plant extract.

In another particular embodiment of the combination of the invention, as defined above, optionally in combination with any of the particular embodiments described above or below throughout the description, the Garcinia cambogia plant extract is an extract prepared from the rind of the dry fruit of a Garcinia cambogia plant.

In another particular embodiment of the combination of the invention, as defined above, optionally in combination with any of the particular embodiments described above or below throughout the description, the plant extract comprises hydroxycitric acid, or the pharmaceutically acceptable or the edible salt thereof, in an amount such that the amount corresponding to hydroxycitric acid is equal to or higher than 30 %w/w of dry extract mass, in particular equal to or higher than 40 %w/w of dry extract mass, in particular equal to or higher than 50 %w/w of dry extract mass, and in particular from 35 to 60 %w/w of dry extract mass.ln another particular embodiment of the combination of the invention, as defined above, optionally in combination with any of the particular embodiments described above or below throughout the description, hydroxycitric acid, or the pharmaceutically acceptable or the edible salt thereof, is hydroxycitric acid which has been extracted from a plant or part of a plant rich in hydroxycitric acid. In yet another particular embodiment, hydroxycitric acid, or the pharmaceutically acceptable or the edible salt thereof, is derived from a plant or part of a plant rich in hydroxycitric acid. In a further particular embodiment, hydroxycitric acid, or the pharmaceutically acceptable or the edible salt thereof, is a pharmaceutically acceptable or edible salt of hydroxycitric acid that is derived from hydroxycitric acid which has been extracted from a plant or part of a plant rich in hydroxycitric acid.

In another particular embodiment of the combination of the invention, as defined above, optionally in combination with any of the particular embodiments described above or below throughout the description, hydroxycitric acid, or the pharmaceutically acceptable or the edible salt thereof, is (-)-hydroxycitric acid which has been extracted from a plant or part of a plant rich in hydroxycitric acid. In yet another particular embodiment, hydroxycitric acid, or the pharmaceutically acceptable or the edible salt thereof, is (-)-hydroxycitric acid derived from a plant or part of a plant rich in hydroxycitric acid. In a further particular embodiment, hydroxycitric acid, or the pharmaceutically acceptable or the edible salt thereof, is a pharmaceutically acceptable or edible salt of (-)-hydroxycitric acid that is derived from (-)-hydroxycitric acid which has been extracted from a plant or part of a plant rich in hydroxycitric acid In another particular embodiment of the combination of the invention, as defined above, optionally in combination with any of the particular embodiments described above or below throughout the description, phytic acid, or the pharmaceutically acceptable or the edible salt thereof, is provided in the form of a plant extract from a plant species or part of a plant species rich in phytic acid or its pharmaceutically acceptable or its edible salts.

In another particular embodiment of the combination of the invention, as defined above, optionally in combination with any of the particular embodiments described above or below throughout the description, phytic acid, or the pharmaceutically acceptable or the edible salt thereof, is provided in the form of a plant extract selected from a group consisting of: a rice bran plant extract, a wheat bran plant extract, a maize germ plant extract, a bean plant extract, and combinations thereof.

In another particular embodiment of the combination of the invention, as defined above, optionally in combination with any of the particular embodiments described above or below throughout the description, phytic acid, or the pharmaceutically acceptable or the edible salt thereof, is provided in the form of a rice bran plant extract.

In another particular embodiment of the combination of the invention, as defined above, optionally in combination with any of the particular embodiments described above or below throughout the description, the rice bran plant extract comprises phytic acid, or the pharmaceutically acceptable or the edible salt thereof, in an amount such that the amount corresponding to the mass of phytic acid is from 0.1 to 10 %w/w of dry extract mass.

In another particular embodiment of the combination of the invention, as defined above, optionally in combination with any of the particular embodiments described above or below throughout the description, phytic acid, or the pharmaceutically acceptable or the edible salt thereof, is phytic acid which has been extracted from a plant or part of a plant rich in phytic acid. In yet another particular embodiment, phytic acid, or the pharmaceutically acceptable or the edible salt thereof, is derived from a plant or part of a plant rich in phytic acid. In a further particular embodiment, phytic acid, or the pharmaceutically acceptable or the edible salt thereof, is a pharmaceutically acceptable or edible salt of phytic acid that is derived from phytic acid which has been extracted from a plant or part of a plant rich in phytic acid.

In another particular embodiment of the combination of the invention, as defined above, optionally in combination with any of the particular embodiments described above or below throughout the description, the plant extract is selected from the group consisting of an aqueous extract, an alcoholic extract, and an organic extract.

The mass amounts, weight ratios and molar ratios of hydroxycitric acid, or of the pharmaceutically acceptable or the edible salt thereof, and of phytic acid, or of the pharmaceutically acceptable or the edible salt thereof, disclosed in the particular embodiments of the invention, above and below throughout the description, were calculated taking into account: (i) normal urinary volume output in adults, (ii) the urinary excretion rates of hydroxycitric acid and of phytic acid, or their respective salts, as disclosed above, and (iii) the molecular weights of hydroxycitric acid and of phytic acid, respectively.

Thus, in another particular embodiment, the combination of the invention, as defined above, optionally in combination with any of the particular embodiments described above or below throughout the description, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in an amount such that the amount administered to a subject corresponds to from 500 - 15,000 mg of hydroxycitric acid in 24 hours, particularly 750 - 10,000 mg of hydroxycitric acid in 24 hours, particularly 1,000 - 5,000 mg of hydroxycitric acid in 24 hours, particularly 1,500 - 3,000 mg of hydroxycitric acid in 24 hours.

In another particular embodiment, the combination of the invention, as defined above, optionally in combination with any of the particular embodiments described above or below throughout the description, comprises phytic acid, or the pharmaceutically acceptable or the edible salt thereof, or, alternatively, the rice bran plant extract which comprises phytic acid, or the pharmaceutically acceptable or the edible salt thereof, in an amount such that the amount administered to a subject corresponds to from 50 - 5,000 mg of phytic acid in 24 hours, particularly 100 - 3,500 mg of phytic acid in 24 hours, particularly 150 - 2,000 mg of phytic acid in 24 hours, particularly 200 - 500 mg of phytic acid in 24 hours.

In another particular embodiment, the combination of the invention, as defined above, optionally in combination with any of the particular embodiments described above or below throughout the description, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in amounts corresponding to from 500 - 15,000 mg of hydroxycitric acid, particularly 750 - 10,000 mg of hydroxycitric acid, particularly 1,000 - 5,000 mg of hydroxycitric acid, particularly 1,500 - 3,000 mg of hydroxycitric acid, wherein said amounts of hydroxycitric acid, or the pharmaceutically acceptable or the edible salt thereof, are comprised in a single dosage form or in multiple dosage forms and are to be administered to a subject over 24 hours.

In another particular embodiment, the combination of the invention, as defined above, optionally in combination with any of the particular embodiments described above or below throughout the description, comprises phytic acid, or the pharmaceutically acceptable or the edible salt thereof, or, alternatively, the rice bran plant extract which comprises phytic acid, or the pharmaceutically acceptable or the edible salt thereof, in amounts corresponding to from 50 - 5000 mg of phytic acid, more particularly 100 - 3,500 mg of phytic acid, more particularly 150 - 2,000 mg of phytic acid, even more particularly 200 - 500 mg of phytic acid, wherein said amounts of phytic acid, or the pharmaceutically acceptable or the edible salt thereof, are comprised in a single dosage form or in multiple dosage forms and are to be administered to a subject over 24 hours.

In a further particular embodiment, the combination of the invention, as defined above, optionally in combination with any of the particular embodiments described above or below throughout the description, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in amounts corresponding to from 1,500 - 3,000 mg of hydroxycitric acid, and comprises phytic acid, or the pharmaceutically acceptable or the edible salt thereof, or, alternatively, the rice bran plant extract which comprises phytic acid, or the pharmaceutically acceptable or the edible salt thereof, in amounts corresponding to from 200 - 500 mg of phytic acid, wherein said amounts of hydroxycitric acid, or the pharmaceutically acceptable or the edible salt thereof, and of phytic acid, or the pharmaceutically acceptable or the edible salt thereof, are comprised, together or separately, in a single dosage form or in multiple dosage forms and are to be administered to a subject over 24 hours.

In another particular embodiment, the combination of the invention, as defined above, optionally in combination with any of the particular embodiments described above or below throughout the description, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in an amount such that the amount administered to a subject corresponds to from 5 - 3,000 mg of hydroxycitric acid per kg of body weight in 24 hours, particularly 6 - 1,000 mg of hydroxycitric acid per kg of body weight in 24 hours, particularly 7 - 500 mg of hydroxycitric acid per kg of body weight in 24 hours, particularly 8 - 200 mg of hydroxycitric acid per kg of body weight in 24 hours, particularly 9 - 100 mg of hydroxycitric acid per kg of body weight in 24 hours, particularly 10 - 50 mg of hydroxycitric acid per kg of body weight in 24 hours.

In another particular embodiment, the combination of the invention, as defined above, optionally in combination with any of the particular embodiments described above or below throughout the description, comprises phytic acid, or the pharmaceutically acceptable or the edible salt thereof, or, alternatively, the rice bran plant extract which comprises phytic acid, or the pharmaceutically acceptable or the edible salt thereof, in an amount such that the amount administered to a subject corresponds to from 1 - 100 mg of phytic acid per kg of body weight in 24 hours, particularly 2 - 80 mg of phytic acid per kg of body weight in 24 hours, particularly 3 - 60 mg of phytic acid per kg of body weight in 24 hours, particularly 4 - 40 mg of phytic acid per kg of body weight in 24 hours, particularly 5 - 20 mg of phytic acid per kg of body weight in 24 hours, particularly 6-10 mg of phytic acid per kg of body weight in 24 hours.

Also part of the invention is a combination comprising hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, as defined above, for use in the treatment and/or prevention of at least one condition characterised by the pathological deposition of calcium oxalate crystals in a mammal, wherein the treatment comprises administering a therapeutically effective amount of the combination.

This aspect may also be defined as use of the combination comprising hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, as defined above, for the preparation of a medicament or for the manufacture of foodstuff, drinks or animal feed for the treatment and/or prevention of at least one condition characterised by the pathological deposition of calcium oxalate crystals in a mammal.

Similarly, the present invention provides a method for the treatment and/or prevention of conditions characterised by the pathological deposition of calcium oxalate crystals, wherein said method comprises administering hydroxycitric acid, or a pharmaceutically acceptable or an edible salt thereof, or, alternatively, a Garcinia plant extract which comprises hydroxycitric acid, or a pharmaceutically acceptable or an edible salt thereof, and phytic acid, or a pharmaceutically acceptable or an edible salt thereof, or, alternatively, a rice bran plant extract which comprises phytic acid, or a pharmaceutically acceptable or an edible salt thereof, together with pharmaceutical excipients or carriers, to a subject in need of thereof.

In a particular embodiment of the combination for use or of the method for treatment and/or prevention, as defined above, optionally in combination with any of the particular embodiments described above or below throughout the description, hydroxycitric acid, or the pharmaceutically acceptable or the edible salt thereof, or, alternatively, the Garcinia plant extract which comprises hydroxycitric acid, or the pharmaceutically acceptable or the edible salt thereof, and phytic acid, or the pharmaceutically acceptable or the edible salt thereof, or, alternatively, the rice bran plant extract which comprises phytic acid, or the pharmaceutically acceptable or the edible salt thereof, are administered simultaneously, sequentially in any order, or separately in any order.

In another particular embodiment of the combination for use or of the method for treatment and/or prevention, as defined above, optionally in combination with any of the particular embodiments described above or below throughout the description, the at least one condition characterised by the pathological deposition of calcium oxalate crystals is lithiasis.

In another particular embodiment of the combination for use or of the method for treatment and/or prevention, as defined above, optionally in combination with any of the particular embodiments described above or below throughout the description, the at least one condition characterised by the pathological deposition of calcium oxalate crystals is characterised by the deposition of calcium oxalate crystals in the urinary tract.

In another particular embodiment of the combination for use or of the method for treatment and/or prevention, as defined above, optionally in combination with any of the particular embodiments described above or below throughout the description, the at least one condition characterised by the pathological deposition of calcium oxalate crystals is renal lithiasis.

In another particular embodiment of the combination for use or of the method for treatment and/or prevention, as defined above, optionally in combination with any of the particular embodiments described above or below throughout the description, the at least one condition characterised by the pathological deposition of calcium oxalate crystals is urolithiasis or nephrolithiasis.

In another particular embodiment, optionally in combination with any of the particular embodiments described above or below throughout the description, the combination for use, as defined above, is administered to a subject diagnosed with a condition selected from: hypocitraturia, hyperoxaluria, hypercalciuria and combinations thereof.

In another particular embodiment, optionally in combination with any of the particular embodiments described above or below throughout the description, the combination for use, as defined above, is administered to a subject diagnosed with hypocitraturia.

In another particular embodiment, optionally in combination with any of the particular embodiments described above or below throughout the description, the combination for use, as defined above, is administered to a subject that has a urinary citrate excretion rate over 24 hours of 550 mg or less, particularly 500 mg or less, particularly 450 mg or less, particularly 400 mg or less, particularly 350 mg or less, particularly 300 mg or less, particularly 250 mg or less, particularly 200 mg or less, particularly 150 mg or less.

In another particular embodiment, optionally in combination with any of the particular embodiments described above or below throughout the description, the combination for use, as defined above, is administered to a subject that has a urinary citrate excretion rate of 220 mg or less over 24 hours.

In another particular embodiment, optionally in combination with any of the particular embodiments described above or below throughout the description, the combination for use, as defined above, is administered to an adult human male subject that has a urinary citrate excretion rate of 115 mg or less over 24 hours, or to an adult human female subject that has a urinary citrate excretion rate of 200 mg or less over 24 hours.

In an alternative particular embodiment, optionally in combination with any of the particular embodiments described above or below throughout the description, the method for treatment and/or prevention, as defined above, comprises administering the combination of the invention, as defined above, to a subject diagnosed with a condition selected from hypocitraturia, hyperoxaluria, hypercalciuria, and combinations thereof. In a particular embodiment, the subject is diagnosed with hypocitraturia.

In another particular embodiment of the combination for use or of the method for treatment and/or prevention, as defined above, optionally in combination with any of the particular embodiments described above or below throughout the description, the mammal is a human.

In another particular embodiment of the combination for use or of the method for treatment and/or prevention, as defined above, optionally in combination with any of the particular embodiments described above or below throughout the description, the mammal is a non-human animal.

In another particular embodiment of the combination for use or of the method for treatment and/or prevention, as defined above, optionally in combination with any of the particular embodiments described above or below throughout the description, the treatment comprises the enteral and/or parenteral administration of the combination of the invention, as defined above.

In another particular embodiment of the combination for use or of the method for treatment and/or prevention, as defined above, optionally in combination with any of the particular embodiments described above or below throughout the description, the treatment comprises the enteral administration of the combination of the invention, as defined above.

In another particular embodiment of the combination for use or of the method for treatment and/or prevention, as defined above, optionally in combination with any of the particular embodiments described above or below throughout the description, the treatment comprises the oral administration of the combination of the invention.

In another particular embodiment of the combination for use or of the method for treatment and/or prevention, as defined above, optionally in combination with any of the particular embodiments described above or below throughout the description, hydroxycitric acid, or the pharmaceutically acceptable or the edible salt thereof, is (-)-hydroxycitric acid, or a pharmaceutically acceptable or an edible salt thereof.

In another particular embodiment of the combination for use or of the method for treatment and/or prevention, as defined above, optionally in combination with any of the particular embodiments described above or below throughout the description, therapeutically effective amounts of hydroxycitric acid, or the pharmaceutically acceptable or the edible salt thereof, or, alternatively, of the Garcinia plant extract which comprises hydroxycitric acid, or the pharmaceutically acceptable or the edible salt thereof, and phytic acid, or the pharmaceutically acceptable or the edible salt thereof, or, alternatively, of the rice bran plant extract which comprises phytic acid, or the pharmaceutically acceptable or the edible salt thereof, are administered to a subject at least once per week.

In a particular embodiment of the combination for use or of the method for treatment and/or prevention, as defined above optionally in combination with any of the particular embodiments described above or below throughout the description, therapeutically effective amounts of hydroxycitric acid, or the pharmaceutically acceptable or the edible salt thereof, or, alternatively, of the Garcinia plant extract which comprises hydroxycitric acid, or the pharmaceutically acceptable or the edible salt thereof, and phytic acid, or the pharmaceutically acceptable or the edible salt thereof, or, alternatively, of the rice bran plant extract which comprises phytic acid, or the pharmaceutically acceptable or the edible salt thereof, are administered to a subject for at least 10 days, particularly at least 30 days, particularly at least 90 days, particularly at least 6 months, particularly at least 12 months.

Hydroxycitric acid, or a pharmaceutically acceptable or an edible salt thereof, or, alternatively, a Garcinia plant extract which comprises hydroxycitric acid, or a pharmaceutically acceptable or an edible salt thereof, for use in the treatment and/or prevention of at least one condition characterised by the pathological deposition of calcium oxalate crystals in a mammal, in combination therapy with phytic acid, or a pharmaceutically acceptable or an edible salt thereof, or, alternatively, with a rice bran plant extract which comprises phytic acid, or a pharmaceutically acceptable or an edible salt thereof, wherein the treatment comprises administering hydroxycitric acid, or the pharmaceutically acceptable or the edible salt thereof, and phytic acid, or the pharmaceutically acceptable or the edible salt thereof, in a molar ratio from 10:1 to 800:1, is also part of the invention.

In a particular embodiment of hydroxycitric acid, or the pharmaceutically acceptable or the edible salt thereof, or, alternatively, a Garcinia plant extract which comprises hydroxycitric acid, or a pharmaceutically acceptable or an edible salt thereof, for use in the treatment and/or prevention of at least one condition characterised by the pathological deposition of calcium oxalate crystals in a mammal, as defined above, optionally in combination with any of the particular embodiments described above or below throughout the description, hydroxycitric acid, or the pharmaceutically acceptable or the edible salt thereof, is (-)-hydroxycitric acid, or a pharmaceutically acceptable or an edible salt thereof, or, alternatively, the Garcinia plant extract which comprises hydroxycitric acid, or the pharmaceutically acceptable or the edible salt thereof, is a Garcinia plant extract which comprises (-)-hydroxycitric acid, or a pharmaceutically acceptable or an edible salt thereof. In a particular embodiment of hydroxycitric acid, or the pharmaceutically acceptable or the edible salt thereof, or, alternatively, a Garcinia plant extract which comprises hydroxycitric acid, or a pharmaceutically acceptable or an edible salt thereof, for use in the treatment and/or prevention of at least one condition characterised by the pathological deposition of calcium oxalate crystals in a mammal, as defined above, optionally in combination with any of the particular embodiments described above or below throughout the description, the mammal has been diagnosed with a condition selected from a list consisting of: hypocitraturia, hyperoxaluria, hypercalciuria, and combinations thereof. In a further particular embodiment, the mammal has been diagnosed with hypocitraturia. In an alternative further particular embodiment, the mammal has a urinary citrate excretion rate of 220 mg or less over 24 hours.

Phytic acid, or a pharmaceutically acceptable or an edible salt thereof, or, alternatively, a rice bran plant extract which comprises phytic acid, or a pharmaceutically acceptable or an edible salt thereof, for use in the treatment and/or prevention of at least one condition characterised by the pathological deposition of calcium oxalate crystals in a mammal, in combination therapy with hydroxycitric acid, or a pharmaceutically acceptable or an edible salt thereof, or, alternatively, a Garcinia plant extract which comprises hydroxycitric acid, or a pharmaceutically acceptable or an edible salt thereof, wherein the treatment comprises administering hydroxycitric acid, or the pharmaceutically acceptable or the edible salt thereof, and phytic acid, or the pharmaceutically acceptable or the edible salt thereof, in a molar ratio from 10:1 to 800:1, is also part of the invention.

In a particular embodiment of phytic acid, or the pharmaceutically acceptable or the edible salt thereof, or, alternatively, the rice bran plant extract which comprises phytic acid, or the pharmaceutically acceptable or the edible salt thereof, for use in the treatment and/or prevention of at least one condition characterised by the pathological deposition of calcium oxalate crystals in a mammal, as defined above, optionally in combination with any of the particular embodiments described above or below throughout the description, the hydroxycitric acid, or the pharmaceutically acceptable or the edible salt thereof, is (-)-hydroxycitric acid, or a pharmaceutically acceptable or an edible salt thereof, or, alternatively the Garcinia plant extract which comprises hydroxycitric acid, or the pharmaceutically acceptable or the edible salt thereof, is a Garcinia plant extract which comprises (-)-hydroxycitric acid, or a pharmaceutically acceptable or an edible salt thereof.

In a particular embodiment of phytic acid, or the pharmaceutically acceptable or the edible salt thereof, or, alternatively, the rice bran plant extract which comprises phytic acid, or the pharmaceutically acceptable or the edible salt thereof, for use in the treatment and/or prevention of at least one condition characterised by the pathological deposition of calcium oxalate crystals in a mammal, as defined above, optionally in combination with any of the particular embodiments described above or below throughout the description, the mammal has been diagnosed with a condition selected from a list consisting of: hypocitraturia, hyperoxaluria, hypercalciuria, and combinations thereof. In a further particular embodiment, the mammal has been diagnosed with hypocitraturia. In an alternative further particular embodiment, the mammal has a urinary citrate excretion rate of 220 mg or less over 24 hours.

A pharmaceutical composition comprising a therapeutically effective amount of the combination of the invention, as defined above, together with pharmaceutical excipients or carriers, is also part of the invention.

In a particular embodiment, optionally in combination with any of the particular embodiments described above or below throughout the description, the pharmaceutical composition of the invention, as defined above, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in amounts corresponding to from 1,500 - 4,000 mg of hydroxycitric acid, particularly 1,700 - 3,000 mg of hydroxycitric acid, more particularly 1,800 - 2,500 mg of hydroxycitric acid, most particularly 1,900 - 2,300 mg of hydroxycitric acid.

In a particular embodiment, optionally in combination with any of the particular embodiments described above or below throughout the description, the pharmaceutical composition of the invention, as defined above, comprises phytic acid, or the pharmaceutically acceptable or the edible salt thereof, or, alternatively, rice bran plant extract which comprises phytic acid, or the pharmaceutically acceptable or the edible salt thereof, in amounts corresponding to from 250 - 450 mg of phytic acid acid, particularly 260 - 400 mg of phytic acid acid, more particularly 270 - 375 mg of phytic acid acid, most particularly 280 - 350 mg of phytic acid acid.

In a particular embodiment, optionally in combination with any of the particular embodiments described above or below throughout the description, the pharmaceutical composition of the invention, as defined above, comprises hydroxycitric acid, or the pharmaceutically acceptable or the edible salt thereof, or, alternatively, the Garcinia plant extract which comprises hydroxycitric acid, or the pharmaceutically acceptable or the edible salt thereof, in amounts corresponding to from 1,800 - 2,200 mg of hydroxycitric acid, and comprises phytic acid, or the pharmaceutically acceptable or the edible salt thereof, or, alternatively, rice bran plant extract which comprises phytic acid, or the pharmaceutically acceptable or the edible salt thereof, in amounts corresponding to from 275 - 325 mg of phytic acid acid.

In a particular embodiment, optionally in combination with any of the particular embodiments described above or below throughout the description, the pharmaceutical composition of the invention, as defined above, is formulated as a single combined dosage form for simultaneous administration of the combination of the invention.

In a particular embodiment, optionally in combination with any of the particular embodiments described above or below throughout the description, the pharmaceutical composition of the invention, as defined above, is formulated as a liquid, solid, semi-solid, powder or gel dosage form.

In a particular embodiment, optionally in combination with any of the particular embodiments described above or below throughout the description, the pharmaceutical composition of the invention, as defined above, is administered via the enteral or parenteral route.

In a particular embodiment, optionally in combination with any of the particular embodiments described above or below throughout the description, the pharmaceutical composition of the invention, as defined above, is administered via the enteral route.

In a particular embodiment, optionally in combination with any of the particular embodiments described above or below throughout the description, the pharmaceutical composition of the invention, as defined above, is administered orally.

Also part of the invention is a kit comprising: (a) a pharmaceutical composition comprising a therapeutically effective amount of hydroxycitric acid, or a pharmaceutically acceptable or an edible salt thereof, or, alternatively, a Garcinia plant extract which comprises hydroxycitric acid, or a pharmaceutically acceptable or an edible salt thereof, together with pharmaceutical excipients or carriers; (b) a pharmaceutical composition comprising a therapeutically effective amount of phytic acid, or a pharmaceutically acceptable or an edible salt thereof, or, alternatively, a rice bran plant extract which comprises phytic acid, or a pharmaceutically acceptable or an edible salt thereof, together with pharmaceutical excipients or carriers; and (c) optionally, instructions for their administration; wherein: the amounts to be administered of hydroxycitric acid, or the pharmaceutically acceptable or the edible salt thereof, and of phytic acid, or the pharmaceutically acceptable or the edible salt thereof, are such that the molar ratio of the hydroxycitric acid, or the pharmaceutically acceptable or the edible salt thereof, and the phytic acid, or the pharmaceutically acceptable or the edible salt thereof, is from 10:1 to 800:1.

In a particular embodiment of the kit of the invention, as defined above, optionally in combination with any of the particular embodiments described above or below throughout the description, hydroxycitric acid, or the pharmaceutically acceptable or the edible salt thereof, is (-)-hydroxycitric acid, or a pharmaceutically acceptable or an edible salt thereof, or, alternatively, the Garcinia plant extract which comprises hydroxycitric acid, or the pharmaceutically acceptable or the edible salt thereof, is a Garcinia plant extract which comprises (-)-hydroxycitric acid, or a pharmaceutically acceptable or an edible salt thereof.

In another particular embodiment of the kit of the invention, as defined above, optionally in combination with any of the particular embodiments described above or below throughout the description, the pharmaceutical composition comprising the therapeutically effective amount of hydroxycitric acid, or the pharmaceutically acceptable or the edible salt thereof, or, alternatively, the Garcinia plant extract which comprises hydroxycitric acid, or the pharmaceutically acceptable or the edible salt thereof, together with pharmaceutical excipients or carriers, is administered to a subject simultaneously with, sequentially before, sequentially after or separately from, either before or after, the pharmaceutical composition comprising the therapeutically effective amount of phytic acid, or the pharmaceutically acceptable or the edible salt thereof, or, alternatively, the rice bran plant extract which comprises phytic acid, or the pharmaceutically acceptable or the edible salt thereof, together with pharmaceutical excipients or carriers.

In another particular embodiment, optionally in combination with any of the particular embodiments described above or below throughout the description, the kit of the invention, as defined above, comprises a first composition comprising hydroxycitric acid, or the pharmaceutically acceptable or the edible salt thereof, or, alternatively, the Garcinia plant extract which comprises hydroxycitric acid, or the pharmaceutically acceptable or the edible salt thereof, formulated as a first dosage form, and a second composition comprising phytic acid, or the pharmaceutically acceptable or the edible salt thereof, or, alternatively, the rice bran plant extract which comprises phytic acid, or the pharmaceutically acceptable or the edible salt thereof, formulated as a second dosage form. The separate dosage forms may be administered to a subject either simultaneously, sequentially in any order or separately in any order.

In another particular embodiment of the kit of the invention, as defined above, optionally in combination with any of the particular embodiments described above or below throughout the description, therapeutically effective amounts of hydroxycitric acid, or of the pharmaceutically acceptable or the edible salt thereof, or, alternatively, of the Garcinia plant extract which comprises hydroxycitric acid, or of the pharmaceutically acceptable or the edible salt thereof, are administered to a subject sequentially with the therapeutically effective amounts of phytic acid, or of the pharmaceutically acceptable or the edible salt thereof, wherein the administration of the therapeutically effective amounts of hydroxycitric acid, or of the pharmaceutically acceptable or the edible salt thereof, or, alternatively, of the Garcinia plant extract which comprises hydroxycitric acid, or the pharmaceutically acceptable or the edible salt thereof, occurs at least 1 hour, particularly at least 2 hours, particularly at least 3 hours, particularly at least 6 hours, before or after the administration of the therapeutically effective amounts phytic acid, or of the pharmaceutically acceptable or the edible salt thereof, or, alternatively, of the rice bran plant extract which comprises phytic acid, or the pharmaceutically acceptable or the edible salt thereof.

In another particular embodiment of the kit of the invention, as defined above, optionally in combination with any of the particular embodiments described above or below throughout the description, the therapeutically effective amounts of hydroxycitric acid, or of the pharmaceutically acceptable or the edible salt thereof, or, alternatively, of the Garcinia plant extract which comprises hydroxycitric acid, or of the pharmaceutically acceptable or the edible salt thereof, and the therapeutically effective amounts of phytic acid, or of the pharmaceutically acceptable or the edible salt thereof, or, alternatively, of the rice bran plant extract which comprises phytic acid, or the pharmaceutically acceptable or the edible salt thereof, are formulated as separate dosage forms, wherein said separate dosage forms are co-packaged in a single container, are co-packaged in a plurality of containers within a single outer package, are co-presented in separate packaging ("common presentation"), or are separately packaged and available for sale independently of one another but are co-marketed or co-promoted.

An edible product comprising a therapeutically effective amount of the combination of the invention, as defined above, is also part of the invention.

In a particular embodiment, optionally in combination with any of the particular embodiments described above or below throughout the description, the edible product of the invention, as defined above, is selected from a foodstuff product, a drink product, a dietary supplement for human consumption, an animal feed product, and a dietary supplement for consumption by non-human mammals.

In another further particular embodiment, optionally in combination with any of the particular embodiments described above or below throughout the description, the edible product of the invention, as defined above, additionally incorporates other active ingredients which reinforce the beneficial effects of the combination of the invention.

The following examples and drawings are provided by way of illustration, and they are not intended to be limiting of the present invention. Furthermore, the present invention covers all possible combinations of particular embodiments described herein.

### Examples

### Materials and Methods:

A turbidimetric assay was carried out to determine the effect of the inhibitors on calcium oxalate crystallisation in a synthetic urine medium at pH 6. The synthetic urine used was composed of two different solutions with the following salt concentrations:

### Solution A:

| **Salt** | **Concentration (g/L)** | **Concentration (mM)** |
|---|---|---|
| Na₂SO₄·10H₂O | 6.23 | 19.34 |

| | | |
|---|---|---|
| MgSO₄·7H₂O | 1.46 | 5.92 |
| NH₄Cl | 4.64 | 86.75 |
| KCl | 12.13 | 162.69 |
| CaCl₂ | 0.4 | 10 |

### Solution B:

| **Salt** | **Concentration (g/L)** | **Concentration (mM)** |
|---|---|---|
| NaH₂PO₄·2H₂O | 2.41 | 15.45 |
| Na₂HPO₄·12H₂O | 5.60 | 15.64 |
| NaCl | 13.05 | 223.31 |

The two solutions must be adjusted to pH 6 with HCl 1 M before use. When the two solutions were mixed in 1:1 proportion at the start of every experiment, urinary conditions were obtained.

Stock solutions of 10 milimolar of hydroxycitric acid (hydroxicitric acid tripotassium salt monohydrate; Toronto Research Chemicals, cat. no. H825080) or tartronic acid (Sigma-Aldrich, cat. no. 86320) and of 1 milimolar phytic acid (phytic acid sodium salt hydrate; Sigma-Aldrich, cat. no. 68388) were prepared by dissolving the adequate quantity of each in solution B.

The turbidimetric system consisted of a photometer (AvaSpec-ULS2048CL-EVO, Avantes, The Netherlands) that was equipped with a fiber-optic light-guide measuring cell that was attached to a light path (2x10 mm) reflector. This instrument was operated in kinetic mode, and absorbance measurements were integrated from 400 to 600 nm. Crystallisation was assessed at 37 degrees Celsius, to replicate the temperature of mammalian urine *in vivo,* with continuous mixing with a magnetic stir bar (250 rpm).

The basal experiment consisted of 25 mL of solution B added to a flask, followed by the addition of 25 mL of solution A, initiating the turbidimetric lecture. At 30 seconds from the addition of solution A, 0.705 mL of a 40 milimolar sodium oxalate solution (di-sodium oxalate; PanReac AppliChem, cat. no. 131706) were added to induce the crystallisation of calcium oxalate. Absorbance was recorded continuously to monitor crystal formation, as the increase in absorbance allows determining when the crystallisation starts. The "induction time" was defined as the time when the absorbance first began to increase. Each experiment was performed in triplicate.

In the experiments with inhibitors, the corresponding volume of the prepared 10 milimolar inhibitor solution were added to the flask to obtain the desired final concentration (from 5 to 1 milimolar), with the corresponding reduction in the volume of added solution B to reach a final volume of 50 mL in every experiment. To evaluate synergistic effects between hydroxycitric acid or tartonic acid and phytic acid, different concentrations of the two compounds were added to the same beaker.

Crystals that formed during these assays were collected by passing the solution through a 0.45 micron filter 30 min after the induction time. They were then dried in a desiccator and examined by scanning electron microscopy (SEM); the crystals were placed on a sample holder, and fixed with an adhesive conductive tape, then observed via SEM (TM4000 Plus II, Hitachi, Tokyo, Japan).

### Example 1: Synergistic effect for combinations of hydroxycitric acid and phytic acid

The study of the inhibitory effect of mixtures of hydroxycitric acid and phytic acid on the formation of calcium oxalate crystals demonstrated the existence of synergistic effects between both inhibitors.

Thus, as can be seen in FIG. 1, the effects of the hydroxycitric acid+phytic acid mixtures did not present additive effects, but rather, for molar ratios of hydroxycitric acid:phytic acid of 500:1, 1,000:1, 2,000:1 and 4,000:1, their effects were superior to the values expected if the two compounds had acted in an additive manner.

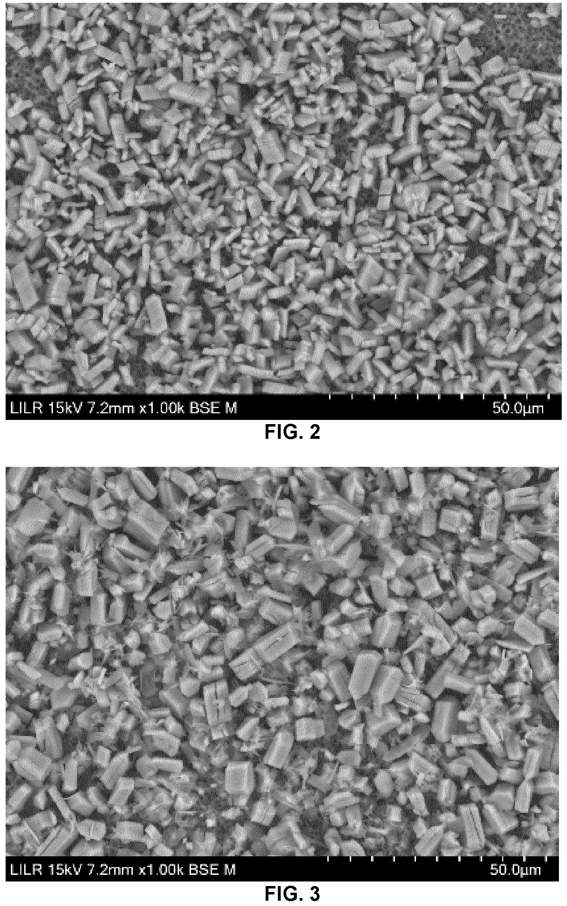

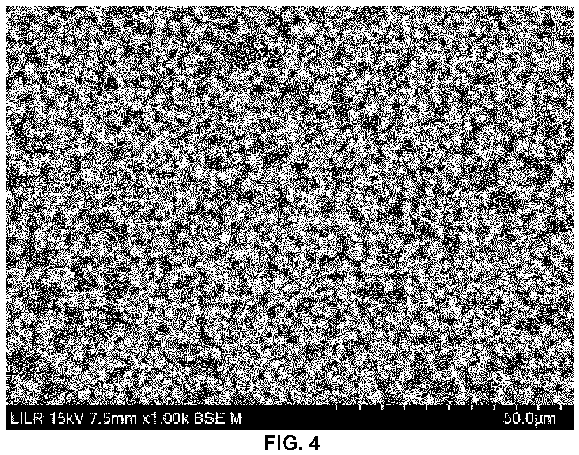

On the other hand, as can be seen in FIGS. 2-4, the calcium oxalate crystals formed in the presence of hydroxycitric acid exclusively or in the presence of phytic acid exclusively, presented a different morphology than those formed in the presence of hydroxycitric acid + phytic acid mixtures, thus confirming these synergistic effects.

### Comparative example 1: No synergistic effect for combinations of tartronic acid and phytic acid

In contrast to the effects observed in the presence of hydroxycitric acid+phytic acid mixtures, no synergistic effects were observed for the combination of phytic acid with another polyhydroxycarboxylic acid, namely tartronic acid, for molar ratios of tartronic acid:phytic acid of 500:1, 1,000:1, 2,000:1 and 4,000:1 (FIG. 5).

Thus, no synergistic effects were observed for the combination of tartronic acid and phytic acid, when the combination of the two compounds was tested using tartronic acid:phytic acid molar ratios of 500:1, 1,000:1, 2,000:1 and 4,000:1, the same molar ratios for which the synergistic effects of the combination of hydroxycitric acid and phytic acid are demonstrated in FIG. 1.

### Citation List

Patent Literature:
WO0000188 A2

### Non-Patent Literature:

Yang et al., "Hydroxycitrate prevents calcium oxalate crystallization and kidney injury in a nephrolithiasis rat model", Urolithiasis, 2022, volume 50, issue 1, pp 47-53
Calvo et al., "Effect of phytic acid (InsP6) and other inositol-phosphates (lnsP5, InsP4, InsP3, InsP2) on crystallization of calcium oxalate, brushite, and hydroxyapatite", Biomolecules, 2023, volume 13, issue 7, pp 1061
Grases et al., "Determination of myo-inositol hexakisphosphate (phytate) in urine by inductively coupled plasma atomic emission spectrometry", 2004, Analytica Chimica Acta, vol. 510, issue 1, pp. 41-43
Grases et al., "Efficacy of mixtures of magnesium, citrate and phytic acid as calcium oxalate crystallization inhibitors in urine", 2015, J Urol, volume 194, issue 3, pp 812-819
Berge et al., "Pharmaceutical Salts", 1997, J Pharm Sci, volume 66, issue 1, pp 1-19
Chung et al., "Molecular modifiers reveal a mechanism of pathological crystal growth inhibition", Nature, 2016, volume 536, pp 446-450
Lewis, "Methods in Enzymology", J. M. Lowenstein, Ed., volume 13, pp. 613-623, Academic Press, New York, 1969
Singh et al., "Extraction and characterization of polyphenolic compounds and potassium hydroxycitrate from Hibiscus sabdariffa", 2021, Future Foods, Volume 4, 100087
Canan et al., "Studies on the extraction and purification of phytic acid from rice bran", J Food Comp Analysis, 2011, volume 24, issue 7, pp 1057-1063

## Claims

1. A combination of:
a) hydroxycitric acid, or a pharmaceutically acceptable or an edible salt thereof, or, alternatively, a Garcinia plant extract which comprises hydroxycitric acid, or a pharmaceutically acceptable or an edible salt thereof,
and
b) phytic acid, or a pharmaceutically acceptable or an edible salt thereof, or, alternatively, a rice bran plant extract which comprises phytic acid, or a pharmaceutically acceptable or an edible salt thereof,
wherein:
the molar ratio of hydroxycitric acid, or the pharmaceutically acceptable or the edible salt thereof, and phytic acid, or the pharmaceutically acceptable or the edible salt thereof, in the combination is from 10:1 to 800:1.

2. The combination according to claim 1, wherein:
the molar ratio of a) and b) in the combination is such that, when the combination is administered to a subject, it results in:
(i) a concentration of phytic acid, or of a pharmaceutically acceptable or an edible salt thereof, in urine from 0.5 micromolar to 5 micromolar of phytic acid, within 20 days post-administration;
and
(ii) a molar ratio of hydroxycitric acid, or the pharmaceutically acceptable or the edible salt thereof, and phytic acid, or the pharmaceutically acceptable or the edible salt thereof, in urine from 500:1 to 8,000:1, expressed as a ratio of the amount, in milimoles, of hydroxycitric acid, or the pharmaceutically acceptable or the edible salt thereof, per litre of urine, to the amount, in milimoles, of phytic acid, or the pharmaceutically acceptable or the edible salt thereof, per litre of urine;
wherein the concentration of hydroxycitric acid, or the pharmaceutically acceptable or the edible salt thereof, in urine is measured by ion chromatography, and the concentration of phytic acid, or the pharmaceutically acceptable or the edible salt thereof, in urine is measured by inductively coupled plasma atomic emission spectrometry.

3. The combination according to any of the claims 1-2, wherein the hydroxycitric acid, or the pharmaceutically acceptable or the edible salt thereof, is (-)-hydroxycitric acid, or a pharmaceutically acceptable or an edible salt thereof.

4. A combination as defined in any of the claims 1-3, for use in the treatment and/or prevention of at least one condition **characterised by** the pathological deposition of calcium oxalate crystals in a mammal, wherein the treatment comprises administering a therapeutically effective amount of the combination.

5. The combination for use according to claim 4, wherein the at least one condition **characterised by** the pathological deposition of calcium oxalate crystals is renal lithiasis.

6. The combination for use according to any of the claims 4-5, wherein the treatment comprises the simultaneous, sequential or separate administration of hydroxycitric acid, or of the pharmaceutically acceptable or the edible salt thereof, or, alternatively, of the Garcinia plant extract which comprises hydroxycitric acid, or the pharmaceutically acceptable or the edible salt thereof, and of phytic acid, or of the pharmaceutically acceptable or the edible salt thereof, or, alternatively, of the rice bran plant extract which comprises phytic acid, or the pharmaceutically acceptable or the edible salt thereof, to a subject in need of thereof.

7. The combination for use according to any of the claims 4-6, wherein the mammal has been diagnosed with a condition selected from: hypocitraturia, hypercalciuria, hyperoxaluria, and combinations thereof.

8. Hydroxycitric acid, or a pharmaceutically acceptable or an edible salt thereof, or, alternatively, a Garcinia plant extract which comprises hydroxycitric acid, or a pharmaceutically acceptable or an edible salt thereof, for use in the treatment and/or prevention of at least one condition **characterised by** the pathological deposition of calcium oxalate crystals in a mammal, in combination therapy with phytic acid, or a pharmaceutically acceptable or an edible salt thereof, or, alternatively, with a rice bran plant extract which comprises phytic acid, or a pharmaceutically acceptable or an edible salt thereof, wherein the treatment comprises administering a molar ratio from 10:1 to 800:1 of hydroxycitric acid, or of the pharmaceutically acceptable or the edible salt thereof, and phytic acid, or the pharmaceutically acceptable or the edible salt thereof.

9. Hydroxycitric acid, or the pharmaceutically acceptable or the edible salt thereof, or, alternatively, the Garcinia plant extract which comprises hydroxycitric acid, or the pharmaceutically acceptable or the edible salt thereof, for use according to claim 8, wherein hydroxycitric acid, or the pharmaceutically acceptable or the edible salt thereof, is (-)-hydroxycitric acid, or a pharmaceutically acceptable or an edible salt thereof.

10. Phytic acid, or a pharmaceutically acceptable or an edible salt thereof, or, alternatively, a rice bran plant extract which comprises phytic acid, or a pharmaceutically acceptable or an edible salt thereof, for use in the treatment and/or prevention of at least one condition **characterised by** the pathological deposition of calcium oxalate crystals in a mammal, in combination therapy with hydroxycitric acid, or a pharmaceutically acceptable or an edible salt thereof, or, alternatively, a Garcinia plant extract which comprises hydroxycitric acid, or a pharmaceutically acceptable or an edible salt thereof, wherein the treatment comprises administering a molar ratio from 10:1 to 800:1 of hydroxycitric acid, or of the pharmaceutically acceptable or the edible salt thereof, and phytic acid, or the pharmaceutically acceptable or the edible salt thereof.

11. Phytic acid, or the pharmaceutically acceptable or the edible salt thereof, or, alternatively, the rice bran plant extract which comprises phytic acid, or the pharmaceutically acceptable or the edible salt thereof, for use according to claim 10, wherein hydroxycitric acid, or the pharmaceutically acceptable or the edible salt thereof, is (-)-hydroxycitric acid, or a pharmaceutically acceptable or an edible salt thereof.

12. A pharmaceutical composition comprising a therapeutically effective amount of the combination of any of the claims 1-4, together with pharmaceutical excipients or carriers.

13. An edible product comprising a therapeutically effective amount of the combination of any of the claims 1-4.

14. A kit comprising:
a) a pharmaceutical composition comprising a therapeutically effective amount of hydroxycitric acid, or a pharmaceutically acceptable or an edible salt thereof, or, alternatively, of a Garcinia plant extract which comprises hydroxycitric acid, or a pharmaceutically acceptable or an edible salt thereof, together with pharmaceutical excipients or carriers;
b) a pharmaceutical composition comprising a therapeutically effective amount of phytic acid, or a pharmaceutically acceptable or an edible salt thereof, or, alternatively, of a rice bran plant extract which comprises phytic acid, or a pharmaceutically acceptable or an edible salt thereof, together with pharmaceutical excipients or carriers; and
c) optionally, instructions for their administration;
wherein:
the amounts to be administered of hydroxycitric acid, or the pharmaceutically acceptable or the edible salt thereof, and of phytic acid, or the pharmaceutically acceptable or the edible salt thereof, are such that the molar ratio of the hydroxycitric acid, or the pharmaceutically acceptable or the edible salt thereof, and the phytic acid, or the pharmaceutically acceptable or the edible salt thereof, is from 10:1 to 800:1.

15. The kit according to claim 14, wherein hydroxycitric acid, or the pharmaceutically acceptable or the edible salt thereof, is (-)-hydroxycitric acid, or a pharmaceutically acceptable or an edible salt thereof.
